# EUROPÄISCHE PATENTSCHRIFT

(11) **EP 2 550 383 B1**
(45) Veröffentlichungstag und Bekanntmachung des Hinweises auf die Patenterteilung: **18.06.2014**
(21) Anmeldenummer: 11708268.5
(22) Anmeldetag: 15.03.2011
(51) Int. Cl.: D01F 2/02, D01F 1/10, D06M 15/03

(54) **VERWENDUNG EINER CELLULOSEFASER**
Use of a cellulose fibre
Utilisation d'une fibre de cellulose

(30) Priorität: 25.03.2010 EP 10157765
(43) Veröffentlichungstag der Anmeldung: 30.01.2013
(73) Patentinhaber: Lenzing Aktiengesellschaft, 4860 Lenzing (AT)
(72) Erfinder: REDLINGER, Sigrid, A-4860 Lenzing (AT); KRONER, Gert, A-4860 Lenzing (AT)
(74) Vertreter: Nemec, Harald
(86) Internationale Anmeldenummer: PCT/EP2011/053875
(87) Internationale Veröffentlichungsnummer: WO 2011/117111

(56) Entgegenhaltungen:
- WO-A1-01/55486
- WO-A1-2004/007818
- WO-A1-2009/092121
- AT-U2- 8 388
- US-A- 5 622 666

## Beschreibung

Die Erfindung betrifft die Verwendung von mit Chitosan modifizierten Cellulosefasern.

Unter "mit Chitosan modifizierten Cellulosefasern" werden für die Zwecke der vorliegenden Erfindung Cellulosefasern verstanden, welche ein Chitosan und/oder ein Chitosansalz inkorporiert enthalten und/oder ein Chitosan und/oder ein Chitosansalz an der Oberfläche aufweisen. Fasern, welche Chitosan (oder ein Chitosansalz) an der Oberfläche aufweisen, werden im folgenden auch als "mit Chitosan imprägnierte" Fasern bezeichnet.

Chitosan besteht aus Poly-(1,4)-2-Amino-2-Deoxy-Beta-D-Glucose und wird durch Deacetylierung von Chitin (Poly-(1,4)-2-Acetamid-2-Deoxy-Beta-D-Glucose) hergestellt. Aus Löslichkeitsgründen - Chitin ist unlöslich in Wasser, organischen Lösungsmitteln, verdünnten Säuren und Laugen - hat Chitosan, welches in verdünnten Säuren, wässrigem Methanol und Glycerin löslich ist, die weitaus größere Bedeutung.

Mit Chitosan modifizierte Cellulosefasern sind bekannt (vgl. AT 008 388 U2):
Kommerziell erhältlich sind Viskosefasern mit inkorporiertem Chitin/Chitosan z.B. unter dem Handelsnamen Crabyon (Fa. Omikenshi Co) und Chitopoly (Fa. Fuji Spinning Co). Hergestellt werden diese Fasern beispielsweise, indem Chitosan oder acetyliertes Chitosan in Pulverform mit einer Korngröße kleiner 10 µm in einer Menge von 0,5 bis 2 Gew.% in Wasser dispergiert wird und der Viskosespinnlösung zugegeben wird (US 5,320,903). Dann werden nach dem herkömmlichen Viskose- oder auch Polynosicverfahren Fasern hergestellt. Diabetikersocken, welche Fasern des Handelsnamens "Crabyon" enthalten, sind kommerziell erhältlich.

Weitere Herstellungsverfahren für chitosaninkorporierte Viskosefasern sind in der US-A 5,756,111 (aufwendige Vor- und Nachlöseprozesse bei Tieftemperatur, um alkalische Chitin-Chitosan-Lösungen für die Zugabe zur Viskoselösung zu erhalten), der US-A 5,622,666 (Zugabe von mikrokristallinem Chitosan und einem wasser- und/oder alkalilöslichen natürlichen Polymer, z.B. Natriumalginat, welches ionische Bindungen zum Chitosan bilden kann, als Dispersion zur Viskosespinnlösung) und PCT/FI90/00292 bzw. FI 78127 (Zugabe von mikrokristallinem Chitosan zur Spinnmasse) beschrieben.

In der DE 195 44 097 wird ein Verfahren zur Herstellung von Formkörpern aus Polysaccharidmischungen durch Auflösen von Cellulose und einem Zweitpolysaccharid in einem organischen, mit Wasser mischbaren Polysaccharidlösungsmittel (bevorzugt NMMO), das auch ein Zweitlösungsmittel enthalten kann, beschrieben.

Weiters wird in der KR-A 9614022 die Herstellung von Chitin-Cellulose-Fasern, genannt "Chitulose", beschrieben, indem Chitin und Cellulose in einem Lösungsmittel aus der Gruppe Dimethylimidazoline/LiCl, Dichloroacetat/Chlorkohlenwasserstoff, Dimethylacetamid/LiCl, N-Methylpyrrolidon/LiCl gelöst werden und nach dem Nassspinnverfahren Garne hergestellt werden.

In der EP-A 0 883 645 wird u.a. die Zugabe von Chitosan zur Lösung als modifizierte Verbindung zur Erhöhung der Geschmeidigkeit von Nahrungsmittelhüllen beansprucht. Die modifizierenden Verbindungen müssen mit der Cellulose/NMMO/Wasser-Lösung mischbar sein.

Die KR-A-2002036398 beschreibt die Inkorporation von aufwendig herzustellenden Chitosan-Derivaten mit quaternären Ammoniumgruppen in Fasern.

In der DE-A 100 07 794 wird die Herstellung von Polymerzusammensetzungen beschrieben, umfassend ein biologisch abbaubares Polymer und ein Material aus Meerespflanzen und/oder Schalen von Meerestieren sowie die Herstellung von Formkörpern daraus. Beansprucht wird auch die Zugabe von Material aus Meerespflanzen, Meerestieren in Pulverform, Pulversüspension oder flüssiger Form zur nach dem Lyocellverfahren hergestellten Celluloselösung.

Weiters wird im Schlussbericht "Erzeugnisse aus Polysaccharidverbunden" (Taeger, E.; Kramer, H.; Meister, F.; Vorwerg, W.; Radosta, S; TITK - Thüringisches Institut für Textil-und Kunststoff-Forschung, 1997, S.1-47, Report-Nr. FKZ 95/NR 036 F) beschrieben, dass Chitosan in verdünnten organischen oder anorganischen Säuren gelöst wird und danach in wässriger NMMO-Lösung gefällt wird. Man erhält so eine Suspension feiner Chitosankristalle in der Celluloselösung, die dann versponnen wird.

Weitere Verfahren zur Herstellung von mit Chitosan modifizierten Cellulosefasern sind in der WO 2004/007818, der WO 2009/092121 sowie der PCT/AT2009/000334 (nicht vorveröffentlicht) beschrieben.

Die EP 1 243 688 beschreibt die Verwendung von Chitosan-Nanopartikeln zur Herstellung von Fasern, Garnen, Gewirken und textilen Flächengebilden. Unter Nano-Chitosanen werden annähernd sphärische Festkörper verstanden, die einen mittleren Durchmesser im Bereich von 10 bis 300 nm aufweisen und aufgrund des kleinen Teilchendurchmessers zwischen die Fibrillen eingelagert werden. Die Herstellung von Nano-Chitosanen erfolgt durch Sprühtrocknung, Evaporationstechnik oder Entspannung von überkritischen Lösungen.

In der WO 01/32751 wird ein Verfahren zur Herstellung von nanopartikulärem Chitosan für kosmetische und pharmazeutische Zubereitungen mit Teilchendurchmessern von 10 bis 1000 nm beschrieben, bei welchem man den pH-Wert einer wässrigen sauren Chitosanlösung in Gegenwart eines Oberflächenmodifikationsmittels soweit anhebt, dass es zur Ausfällung des Chitosans kommt. Weiters wird in der WO 91/00298 die Herstellung von mikrokristallinen Chitosan-Dispersionen und Pulvern mit Teilchendurchmessern von 0,1 bis 50 µm beschrieben, bei welchem man den pH-Wert einer wässrigen sauren Chitosanlösung so weit anhebt, dass es zur Ausfällung des Chitosans kommt.

Die WO 97/07266 beschreibt die Behandlung einer Lyocellfaser mit einer 0,5%igen essigsauren Chitosanlösung.

AT 8388 U beschreibt die Verwendung einer Cellulosefaser, welche ein Chitosan oder ein Chitosansalz inkorporiert und/oder an der Oberfläche der Faser aufweist, in einem nichtgewebten Textil und/oder einem saugfähigen Hygieneprodukt.

Es ist bekannt, textile Artikel mit funktionalen Wirkstoffen, z.B. kosmetischen Agentien, zu versehen.

Zu den hier vorgeschlagenen Maßnahmen gehört das Einbringen von Mikrokapseln, welche einen Wirkstoff enthalten, in den textilen Artikel.

Die WO 09/124686 A beschreibt die Modifizierung eines textilen Artikels mit sogenannten "Mikrosphären", welche im Unterschied zu Mikrokapseln bei Gebrauch nicht zerstört werden und mit dem Wirkstoff "nachgeladen" können werden sollen.

Die DE 199 40 239 A beschreibt einen Pflege und/oder Heilverband, an dessen Innenseite eine therapeutisch wirksame Auflage austauschfähig fixierbar ist.

Weitere mit Wirkstoffen oder kosmetischen Mitteln behandelte textile Artikel werden beispielsweise in der US 2004/082239A, der WO 06/106546 A und der WO 06/068418 A beschrieben.

Bei den bislang vorgeschlagenen solcherart modifizierten Textilien ist aber bislang eine tatsächlich positive Wirkung nicht nachgewiesen worden bzw. weisen die Textilien den Nachteil auf, dass die Wirksamkeit, d.h. die Funktionalität des eingebrachten Wirkstoffes regelmäßig wiederhergestellt ("nachgeladen") werden muss.

Es ist dabei Stand der Technik, im medizinischen bzw. kosmetischen Bereich den Hautzustand durch folgende Messparameter zu charakterisieren: Hautfeuchte, Transepidermaler Wasserverlust, Hautelastizität, Hautoberflächenprofil, Hautrauhigkeit, Faltentiefe, Mikroskopische Beurteilung, pH Wert der Haut, Hautfloraabstriche. Diese Parameter werden z.B. gemessen, um den Effekt einer kosmetischen Behandlung beurteilen zu können.

Insbesondere für die Parameter Hautfeuchte und Transepidermaler Wasserverlust sind Bereiche zur Beurteilung des Hautzustandes wie folgt festgelegt:

| Hautfeuchte in % | Hautzustand |
|---|---|
| < 30 % | Sehr trocken |
| 30 - 40% | Trocken |
| >40% | Normal |
| > 60 % | Geschädigt durch Okklusion, Aufquellen der Hornschicht |

| Transepidermaler Wasserverlust g/m2/h | |
|---|---|
| 3-9 | Normale Haut mit normaler funktionierender Barrierefunktion |
| 10 - 18 | Leicht geschädigte Haut |
| > 25 | Geschädigte Haut mit eindeutig geschädigter Barrierefunktion |

Ein besonderes Bedürfnis besteht hier im Bereich von körpernah, insbesondere hauteng und mit einem gewissen Auflagedruck auf die Haut getragenen, elastischen und ggf. auch körperformenden Textilien. Es ist bekannt, dass körpernah getragene Textilien Einfluss auf die Hautparameter haben. Durch das Bedecken der Haut mit Textilien unter normalen Tragebedingungen nimmt die Hautfeuchte tendentiell ab und der Transepidermale Wasserverlust steigt. Bekannt ist, dass Textilien hergestellt aus Fasern, welche geringe Wasseraufnahmekapazität/ Wasserdampfdurchlässigkeit haben, wie z.B. Polyester, eine sogenannte Okklusion bewirken. Das bedeutet die Blockierung der Abgabe von Wasser und Wärme durch die Haut und in Folge das Aufquellen der Hornschicht, wodurch die Hautfeuchte zwar erhöht wird, aber gleichzeitig die Hautbarrierefunktion geschädigt wird und der transepidermale Wasserverlust steigt. Dieser Effekt führt sogar bei anfänglich gesunder Haut zur Schädigung (Irritationsdermatosen) und bei bereits geschädigter oder trockener Haut ist diese Auswirkung noch größer. Idealerweise soll ein körpernah enganliegendes Textil keinen negativen Einfluss auf die oben genannten Hautparameter haben.

Die vorliegende Erfindung stellt sich zur Aufgabe, einen elastischen textilen Artikel zur Verfügung zu stellen, der sich in Gebrauch mit der Haut eines lebenden Säugetieres, und zwar mit einem Druck p mit 0,001 N/cm² ≤ p < 0,24 N/cm² in Kontakt befindet, und welcher einen positiven Einfluss auf die Haut, insbesondere auf Parameter wie z.B. Hautfeuchte und transepidermalen Wasserverlust ausübt.

Diese Aufgabe wird durch die Verwendung einer Cellulosefaser gemäß Anspruch 1 gelöst.

Es hat sich überraschenderweise gezeigt, dass durch Textilien aus mit Chitosan modifizierten Cellulosefasern nicht nur kein negativer Einfluss auf die oben genannten Hautparameter eintritt, sondern vielmehr diese Parameter sogar positiv beeinflusst werden. Es wurde gefunden, dass abhängig vom Ausgangszustand der gesunden Haut beim Tragen eines hautengen Textils, welches mit Chitosan modifizierte Cellulosefasern aufweist, der transepidermale Wasserverlust gleich bleibt bzw. verringert wird; dies bei gleichzeitigem Erhalt der Hautfeuchte und Abnahme der Hautrauhigkeit.

Die Aufrechterhaltung des natürlichen Mikrofeuchtefilms auf der Haut aufgrund der Präsenz der mit Chitosan modifizierten Cellulosefasern bewirkt auch eine Beibehaltung der Elastizität der Haut und eine Verbesserung des mikroskopischen Hautbildes. Weiters wird auch der Hautflorazustand nicht negativ beeinflusst.

Bei geschädigter Haut wird zusätzlich die Zellneubildung gefördert.

Das Ausmaß des vom textilen Artikel ausgeübten Druckes hängt von der textilen Konstruktion bzw. der Art des eingesetzten elastischen Materials ab.

Dieser Druck kann in Anlehnung an die für medizinische Kompressionsstrümpfe bekannten Messmethoden, z.B. Deutsches Institut für Gütesicherung und Kennzeichnung Medical Compression Hosiery Quality Assurance RAL-GZ 387/1, Prüfung am HOSY- Messgerät (Institut Hohenstein), gemessen werden. Alternativ kann man den Druck auch direkt am Menschen mit kommerziell erhältlichen Drucksensoren, z.B. Kikuhime Druckkissensensor der Fa. TT MediTrade ApS oder piezoresistive Drucksonden der Type MCDM, Hersteller MIPM Mammendorf, messen.

In der Dissertation "Produktentwicklung für körpernahe Bekleidung unter Berücksichtigung der textilen Materialeigenschaften" von Dipl.-Ing. Tünde Kirstein, Fakultät Maschinenwesen der Technischen Universität Dresden, werden weitere Eigenschaften von körpernaher Bekleidung sowie Messmethoden beschrieben.

Für den mittleren Druck p des Artikels auf die Haut kann 0,01 N/cm² ≤ p, bevorzugt 0,05 N/cm² ≤ p, besonders bevorzugt 0,1 N/cm² ≤ p gelten.

Die erfindungsgemäß verwendete Cellulosefaser ist eine Lyocellfaser.

Der Gattungsname "Lyocell" wurde von der BISFA (The International Bureau for the Standardisation of Man Made Fibres) vergeben und steht für Cellulosefasern, die aus Lösungen der Cellulose in einem organischen Lösungsmittel hergestellt werden. Bevorzugt werden als Lösungsmittel tertiäre Aminoxide, insbesondere N-methyl-morpholin-N-oxid (NMMO) eingesetzt. Ein Verfahren zur Herstellung von Lyocellfasern ist z.B. in der US-A 4,246,221 beschrieben.

Die Cellulosefaser ist eine Lyocellfaser mit einem Gehalt an Chitosan von 0,2 Gew.% bis 0,6 Gew.%. Es hat sich nämlich herausgestellt, dass eine Lyocellfaser bereits mit einem geringen Gehalt von Chitosan von 0,1% bis 1% eine kosmetische und, wie in der PCT/AT2009/000334 (nicht vorveröffentlicht) beschrieben, zudem auch wundheilungsfördemde Wirkung hat. Allerdings nimmt der positive Einfluss auf die Hautzellen bei einem höheren Gehalt an Chitosan (insbesondere bei Gehalten von deutlich mehr als 1 Gew.%) wieder ab. Überraschenderweise liegt bei einer mit Chitosan modifizierten Lyocellfaser somit bei einem geringen Gehalt von 0,1 Gew.% bis 1 Gew.% ein Synergieeffekt sowohl hinsichtlich der kosmetischen Wirkung, also bei der kosmetischen Behandlung der Haut, als auch hinsichtlich des positiven Einflusses auf die Hautzellen, beispielsweise zur Verbesserung der Wundheilung und Verringerung der Narbenbildung vor.

Die mit Chitosan modifizierte Cellulosefaser kann bevorzugt nach einem der in der WO 2004/007818, der WO 2009/092121 sowie der PCT/AT2009/000334 (nicht vorveröffentlicht) beschriebenen Verfahren hergestellt sein.

Die PCT/AT2009/000334 beschreibt ein Verfahren zur Behandlung eines cellulosischen Formkörpers, wobei der Formkörper mit einer sauren Lösung eines Chitosans in Kontakt gebracht wird, und welches dadurch gekennzeichnet ist, dass das Chitosan einen Deacetylierungsgrad von mindestens 80%, einen Stickstoffgehalt von zumindest 7 Gew.%, bevorzugt zumindest 7,5 Gew.%, ein Gewichtsmittel der Molmasse M_{w} (D) von 10 kDa bis 1000 kDa, bevorzugt 10 kDa bis 160 kDa und eine Viskosität in 1 Gew.%-Lösung in 1 Gew.%-Essigsäure bei 25°C von 1000 mPas oder weniger, bevorzugt 400 mPas oder weniger, insbesondere bevorzugt 200 mPas oder weniger, aufweist. Die Formkörper liegen insbesondere in Form von Fasern und bei der Behandlung bevorzugt in niemals getrockneter Form vor.

Die mit Chitosan modifizierte Cellulosefaser kann in Mischung mit anderen Fasern wie z.B. Baumwolle, Polyester, Elastan und Polyamid vorliegen. Bei elastischen Artikeln ist die Beimischung eines elastischen (Faser-)Materials erforderlich.

Dem Fachmann ist die Herstellung von elastischen Artikeln insbesondere im textilen Bereich bekannt.

Der Artikel kann mehrlagig aufgebaut sein, wobei die mit Chitosan modifizierte Cellulosefaser auf der mit der Haut in Kontakt kommenden Seite des Artikels vorgesehen sein soll.

Die vorliegende Erfindung betrifft auch einen textilen elastischen Artikel gemäß Anspruch 3.

Die obigen Ausführungen hinsichtlich Details des Artikels und der zu verwendenden Cellulosefaser gelten auch für den erfindungsgemäßen Artikel.

Die vorliegende Erfindung betrifft weiters die Verwendung des erfindungsgemäßen Artikels zur kosmetischen Behandlung der Haut sowie zur Wundheilung und Verringerung der Narbenbildung.

### Beispiele:

Vier Probanden trugen eine Woche mind. 10 Stunden über Nacht maßgeschneiderte enganliegende Ärmlinge, welche den Arm vom Handgelenk bis über das Ellbogengelenk bedecken und einen mittleren Druck von 0,02 N/cm² ausübten. Am letzten Tag wurden diese Ärmlinge nicht ausgezogen, sondern erst bei der Messung abgenommen. Die Ärmlinge wurden zwischen dem Tragen nicht gewaschen. Die Probanden durften die Unterarme nicht eincremen und nur mit Wasser waschen. Es wurde jeweils ein Rechts-/Linksvergleich durchgeführt. Die Messregion war die Unterarminnenseite.

Verwendete Materialien Single Jersey aus Polyester (PES) bzw. Lyocellfaser (Marke "Tencel" bzw. Lyocellfaser "Tencel C" (= Lyocellfaser mit 0,4% Chitosanauflage).

| | Links | Rechts |
|---|---|---|
| Proband 1 | Tencel | Tencel C |
| Proband 2 | Tencel | Tencel C |
| Proband 3 | PES | Tencel |

Vor dem Trageversuch wurden Hautfeuchte und Transepidermaler Wasserverlust = TEWL gemessen. Die Messung erfolgt nach 30 min Klimatisierung bei 24°C / 50 % Luftfeuchtigkeit. Weiters wurde ein Hautfloraabstrich auf der Unterarminnenseite durchgeführt.

Nach dem Trageversuch wird der Ärmel im Klimaraum ausgezogen und nach 30minütiger Klimatisierung werden Hautfeuchte und TEWL gemessen sowie ein Hautfloraabstrich auf der Unterarminnenseite durchgeführt.

### Ergebnisse:

| **Proband 1** | **Tencel** | **% Änderung** | **Tencel C** | **% Änderung** |
|---|---|---|---|---|
| TEWL Ausgangswert | 11,95 g/m2/h | | 16,03 g/m2/h | |
| TEWL n.7 Tagen | 14,39 g/m2/h | + 20,4% | 14,44 g/m2/h | -20% |
| | | | | |
| Hautfeuchte | 43,6% | | 36,1% | |
| Hautfeuchte n.7 Tagen | 38,5% | -11,7% | 34,3% | -4,9% |

| **Proband 2** | **Tencel** | **% Änderung** | **Tencel C** | **% Änderung** |
|---|---|---|---|---|
| TEWL Ausgangswert | 10,28 | | 11,38 | |
| TEWL n.7 Tagen | 11,77 | + 14,5% | 11,65 | 0% |
| | | | | |
| Hautfeuchte | 32,8 | | 37,6 | |
| Hautfeuchte n.7 Tagen | 24,6 | -25% | 30,5 | -18,8% |

| **Proband 3** | **Polyester** | **% Änderung** | **Tencel** | **% Änderung** |
|---|---|---|---|---|
| TEWL Ausgangswert | 4,86 | | 5,57 | |
| TEWL n.7 Tagen | 7,3 | + 50 % | 6,23 | +11,8% |
| | | | | |
| Hautfeuchte | 35 | | 34,4 | |
| Hautfeuchte n.7 Tagen | 37,5 | + 7,1 % | 32,3 | - 6,3% |

## Patentansprüche

1. Verwendung einer Cellulosefaser, welche ein Chitosan und/oder ein Chitosansalz inkorporiert enthält und/oder ein Chitosan und/oder ein Chitosansalz an der Oberfläche aufweist, zur Herstellung eines bei Gebrauch mit der Haut eines lebenden Säugetieres mit einem mittleren Druck p mit 0,001 N/cm² ≤ p < 0,24 N/cm² in Kontakt befindlichen elastischen textilen Artikels ausgewählt aus der Gruppe bestehend aus Miederwaren, Bettwäsche, Leintüchern, Nachtwäsche wie z.B. Pyjamas oder Nachthemden, Unterwäsche, Socken, Kopfpolstern, Leggings, Jeans, T-Shirts, Schlafbrillen, körperformenden Bekleidungsstücken, Schuheinlagen, Bandagen, Mull- und elastischen Binden, Stützverbänden und Trikotschlauchverbänden, wobei die Cellulosefaser in zumindest einem Teil jener Stellen des Artikels, die bei Gebrauch mit der Haut in Kontakt sind, vorgesehen ist und wobei die Cellulosefaser eine Lyocellfaser mit einem Gehalt an Chitosan von 0,2 Gew.% bis 0,6 Gew.% ist.

2. Verwendung gemäß Anspruch 1, **dadurch gekennzeichnet, dass** für den mittleren Druck p des Artikels auf die Haut gilt 0,01 N/cm² ≤ p, bevorzugt 0,05 N/cm² ≤ p, besonders bevorzugt 0,1 N/cm² ≤ p.

3. Textiler elastischer Artikel ausgewählt aus der Gruppe bestehend aus Miederwaren, Bettwäsche, Leintüchern, Nachtwäsche wie z.B. Pyjamas oder Nachthemden, Unterwäsche, Socken, Kopfpolstern, Leggings, Jeans, T-Shirts, Schlafbrillen, körperformenden Bekleidungsstücken, Schuheinlagen,Bandagen, Mull- und elastischen Binden, Stützverbänden und Trikotschlauchverbänden zur Kontaktierung der Haut eines lebenden Säugetieres mit einem mittleren Druck p mit 0,001 N/cm² ≤ p < 0,24 N/cm², enthaltend in zumindest einem Teil jener Stellen des Artikels, die bei Gebrauch mit der Haut in Kontakt sind, eine Cellulosefaser, welche ein Chitosan und/oder ein Chitosansalz inkorporiert enthält und/oder ein Chitosan und/oder ein Chitosansalz an der Oberfläche aufweist, wobei die Cellulosefaser eine Lyocellfaser mit einem Gehalt an Chitosan von 0,2 Gew.% bis 0,6 Gew.% ist.

4. Artikel gemäß Anspruch 3, **dadurch gekennzeichnet, dass** für den mittleren Druck p des Artikels auf die Haut gilt 0,01 N/cm² ≤ p, bevorzugt 0,05 N/cm² ≤ p, besonders bevorzugt 0,1 N/cm² ≤ p.

5. Verwendung eines Artikels gemäß einem der Ansprüche 3 oder 4 zur kosmetischen Behandlung der Haut.

6. Verwendung eines Artikels gemäß einem der Ansprüche 3 oder 4 zur Wundheilung und Verringerung der Narbenbildung.

## Claims

1. The use of a cellulose fibre containing incorporated a chitosan and/or chitosan salt and/or having a chitosan and/or chitosan salt on its surface, for the preparation of an elastic textile article, which, when being used, is in contact with the skin of a living mammal with a mean pressure p of 0.001 N/cm² ≤ p < 0.24 N/cm² and which is selected from the group consisting of corsetry, bed linen, sheets, nightwear, e.g. pyjamas or nightdresses, underwear, socks, pillows, leggings, jeans, t-shirts, sleeping masks, body shaping garments, shoe inserts, bandages, mull, elastic bandages, support bandages, and jersey tube bandages, wherein the cellulose fibre is provided at least in a part of those areas of the article that, when being used, are in contact with the skin, and whereby the cellulose fibre is a Lyocell fibre with a content of chitosan of 0.2 w% to 0.6 w%.

2. The use according to claim 1, **characterized in that** for the mean pressure p of the article applied onto the skin the following applies: 0.01 N/cm² ≤ p, preferably 0.05 N/cm² ≤ p, especially preferably 0.1 N/cm² ≤ p.

3. A textile elastic article, selected from the group consisting of corsetry, bed linen, sheets, nightwear, e.g. pyjamas or nightdresses, underwear, socks, pillows, leggings, jeans, t-shirts, sleeping masks, body shaping garments, shoe inserts, bandages, mull, elastic bandages, support bandages, and jersey tube bandages, for contacting the skin of a living mammal with a mean pressure p of 0.001 N/cm² ≤ p < 0.24 N/cm², containing at least in a part of those areas of the article which, when being used, are in contact with the skin, a cellulose fibre containing incorporated a chitosan and/or chitosan salt and/or having a chitosan and/or chitosan salt on its surface, whereby the cellulose fibre is a Lyocell fibre with a content of chitosan of 0.2 w% to 0.6 w%.

4. An article according to claim 3, **characterized in that** for the mean pressure p of the article onto the skin the following applies: 0.01 N/cm² ≤ p, preferably 0.05 N/cm² ≤ p, especially preferably 0.1 N/cm² ≤ p.

5. The use of an article according to any of the claims 3 or 4 for the cosmetic treatment of the skin.

6. The use of an article according to any of the claims 3 or 4 for wound healing and reduction of scarring.

## Revendications

1. Utilisation d'une fibre de cellulose qui contient de façon incorporée une chitosane et/ou un sel de chitosane, et/ou qui comporte à la surface une chitosane et/ou un sel de chitosane, pour la production d'un article textile élastique qui se trouve en contact avec la peau d'un mammifère vivant en utilisation avec une pression moyenne p telle que 0,001 N/cm² ≤ p < 0,24 N/cm², article choisi parmi le groupe comprenant la lingerie fine, le linge de literie, les draps de lit, la lingerie de nuit comme par exemple pyjamas ou chemises de nuit, sous-vêtements, chaussettes, coussinets de tête, leggings, jeans, T-shirts, masques de nuit, articles vestimentaires modelant le corps, semelles orthopédiques, bandages, bandes de gaze et bandes élastiques, bandages de contention et bandages tubulaires en tricot, dans laquelle la fibre de cellulose est présente au moins dans une partie de ceux des emplacements de l'article qui sont en contact avec la peau en utilisation, et dans laquelle la fibre de cellulose est une fibre de lyocell avec une teneur de chitosane de 0,2 % à 0,6 % en poids.

2. Utilisation selon la revendication 1, **caractérisée en ce que** pour la pression moyenne p de l'article sur la peau s'applique la relation 0,01 N/cm² ≤ p, de préférence 0,05 N/cm² ≤ p, de manière particulièrement préférée 0,1 N/cm² ≤ p.

3. Article textile élastique choisi parmi le groupe comprenant la lingerie fine, le linge de literie, les draps de lit, la lingerie de nuit comme par exemple pyjamas ou chemises de nuit, sous-vêtements, chaussettes, coussinets de tête, leggings, jeans, T-shirts, masques de nuit, articles vestimentaires modelant le corps, semelles orthopédiques, bandages, bandes de gaze et bandes élastiques, bandages de contention et bandages tubulaires en tricot, pour venir en contact avec la peau d'un mammifère vivant sous une pression moyenne p telle que 0,001 N/cm² ≤ p < 0,24 N/cm², contenant dans au moins une partie de ceux des emplacements de l'article qui sont en contact avec la peau en utilisation, une fibre de cellulose qui contient de manière incorporée une chitosane et/ou un sel de chitosane, et/ou qui comporte à la surface une chitosane et/ou un sel de chitosane, dans lequel la fibre de cellulose est une fibre lyocell avec une teneur de chitosane de 0,2 % à 0,6 % en poids.

4. Article selon la revendication 3, **caractérisé en ce que** pour la pression moyenne p de l'article sur la peau s'applique la relation 0,01 N/cm² ≤ p, de préférence 0,05 N/cm² ≤ p, de manière particulièrement préférée 0,1 N/cm² ≤ p.

5. Utilisation d'un article selon l'une des revendications 3 ou 4 pour le traitement cosmétique de la peau.

6. Utilisation d'un article selon l'une des revendications 3 ou 4 pour la cicatrisation de blessures et la réduction de la formation de cicatrices.
